# EUROPÄISCHE PATENTANMELDUNG

(11) **EP 3 162 791 A1**
(43) Veröffentlichungstag der Anmeldung: **03.05.2017**
(21) Anmeldenummer: 15192307.5
(22) Anmeldetag: 30.10.2015
(51) Int. Cl.: C07C 213/00, C07C 215/48

(54) **VERBESSERTES HERSTELLUNGSVERFAHREN FÜR ISOPHORONAMINOALKOHOL (IPAA)**

(71) Anmelder: Evonik Degussa GmbH, 45128 Essen (DE)
(72) Erfinder: Rüfer, Alexander Martin, 45657 Recklinghausen (DE); Rittsteiger, Anne, 59399 Olfen (DE); Spyrou, Emmanouil, 46514 Schermbeck (DE); Schneider, Sven, 45711 Datteln (DE); Sowka, Sabrina, 48249 Dülmen (DE); Ott, Denise, 45772 Marl (DE)

(57) **Zusammenfassung**

Vorliegende Erfindung betrifft ein verbessertes Verfahren zur Herstellung von 3-(Aminomethyl)-3,5,5-trimethylcyclohexanol, im folgenden Isophoronaminoalkohol (IPAA) genannt.

## Beschreibung

Vorliegende Erfindung betrifft ein verbessertes Verfahren zur Herstellung von 3-(Aminomethyl)-3,5,5-trimethylcyclohexanol aus Isophoronnitril, im folgenden Isophoron-aminoalkohol (IPAA) genannt.

IPAA stellt ein wichtiges Zwischenprodukt in verschiedenen Anwendungsbereichen dar. So dient es beispielsweise als Vorstufe pharmakologischer Produkte, insbesondere im Bereich der Influenza-Prophylaxe (WO2011-095576). Weitere Anwendungen beinhalten u.a. den Einsatz in Polymeren, Korrosionschutzmittel und Stabilisatoren (DE1229078).

Die bisherigen Verfahren zeichnen sich dabei durch vergleichsweise komplizierte Reaktionsführung und niedrige Ausbeuten aus.

Pandey et al. (Indian Journal of Chemistry, Vol. 43B, 2004, 2705-2707) beschreibt eine Syntheseroute zu IPAA, ausgehend von Isophoronnnitril (IPN). Dabei wird über mehrere Stufen vorgegangen: Zunächst erfolgt die Bildung des korrespondierenden Amids über Hydrolyse unter basischen Bedingungen. Anschließend erfolgen die Reduktion der Ketofunktion mittels Cyanoborhydrids und die Reduktion des Amids mittels Lithiumaluminiumhydrid.

Eine ähnliche Vorgehensweise ist in den Anmeldungen WO2011/094953 und WO2011/095576 beschrieben, hier erfolgt die Reduktion der Keto- und Nitrilgruppe von IPN jedoch ausschließlich mit Lithiumaluminiumhydrid in THF unter Rückfluss. Als nachteilig ist anzusehen, dass die o.g. Verfahren den stöchiometrischen Einsatz aufwendig herzustellender und damit teurer Reduktionsmittel vorsehen. Weiterhin erfolgt die Synthese über mehrere Stufen und ist damit aufwendig.

Die Anmeldung DE1229078 beschreibt die Herstellung von IPAA aus IPN unter Zuhilfenahme von Ammoniak und Wasserstoff an einem Fischer-Tropsch-Katalysator und sehr hohem Wasserstoffpartialdruck. Die hierbei erforderlichen, relativ hohen Reaktionstemperaturen begünstigen jedoch im Falle von gamma-Ketonitrilen (wie IPN) die Abspaltung von Blausäure (HCN). Dieser Umstand führt zur Ausbeuteminderung und ist daher nachteilig. Als weiterer Nachteil ist anzusehen, dass HCN stark toxisch auf Organismen wirkt (Gefahr bei Freisetzung) und üblicherweise zur Katalysatordesaktivierung beiträgt. Ferner erfolgt substanzielle Dimerenbildung aufgrund der Reaktion der entstehenden Amingruppe mit noch nicht vollständig reduzierter Ketogruppe. Dies stellt ebenso einen Ausbeuteverlust bezogen auf das Wunschprodukt IPAA dar.

Es ist auch bekannt aus EP 42 119, EP 659 734, EP 503 246, WO 2012076315, dass sich bei der konventionellen Hydrierung von IPN, das IPAA nur als Nebenprodukt in unerwünschter Weise bildet, was auch als Nachteil bezeichnet wird. Auch hier wird auf die Abspaltung von Blausäure (HCN) hingewiesen.

Daher sind im Stand der Technik keine geeigneten Katalysatoren zur gezielten einfachen Herstellung von IPAA aus IPN bekannt.

Aufgabe war es deshalb, ein neues verbessertes Verfahren zur gezielten Herstellung von 3-(Aminomethyl)-3,5,5-trimethylcyclohexanol, durch Hydrierung von Isophoronnnitril (IPN) mit Hilfe von Wasserstoff an einem Katalysator zu finden. Das Verfahren sollte sich durch eine einfache Durchführung und einer hohen Ausbeute auszeichnen. Außerdem muss die Abspaltung von Blausäure (HCN) vermieden werden.

Überraschenderweise wurde nun gefunden, dass eine einstufige Hydrierung von IPN mit Hilfe von Wasserstoff an einem heterogenen Katalysator mit einer einfachen Fahrweise und einer sehr hohen Ausbeute möglich ist.

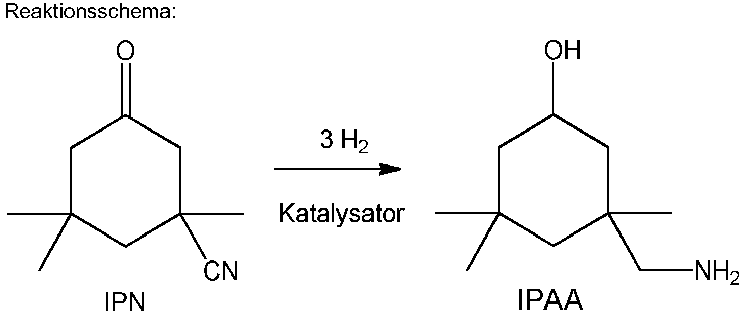

Gegenstand der Erfindung ist ein Verfahren einstufigen zur Herstellung von 3-(Aminomethyl)-3,5,5-trimethylcyclohexanol, durch Hydrierung von Isophoronnnitril (IPN) mit Wasserstoff an einem Katalysator, in An- oder Abwesenheit von Lösungsmittel,
wobei der Katalysator die folgenden Eigenschaften aufweist:
I. Der Katalysator weist nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle:

| | |
|---|---|
| Cobalt: | 55 bis 95 Gew.-% |
| Aluminium: | 5 bis 45 Gew.-% |
| Chrom: | 0 bis 3 Gew.-% |
| Nickel: | 0 bis 7 Gew.-% |

und
II. Der Katalysator liegt in Form von unregelmäßigen Partikeln als Granulat vor und weist nach der Aktivierung Partikelgrößen von 1 bis 8 Millimeter (mm) auf.

### Katalysator

Der Katalysator besteht aus einer Metall-Legierung, wobei die Metall-Legierung durch Basen auf der Oberfläche aktiviert ist. Die Schichtdicke der aktivierten Schicht auf der Partikeloberfläche des Katalysators beträgt bevorzugt 50 bis 1000 Mikrometer (µm). Sie kann aber auch größer oder kleiner sein. Auf der Oberfläche befindet sich demnach die katalytisch aktive Zusammensetzung des Katalysators. Es ist aber auch im Rahmen der Erfindung möglich, das gesamte Katalysator-Partikel fast vollständig oder vollständig auszulaugen.

Der erfindungsgemäße Katalysator liegt nach der Aktivierung als Granulat in Form einzelner Partikel vor.

Der erfindungsgemäße Katalysator weist nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle:
1. Variante

| | |
|---|---|
| Cobalt: | 55 bis 95 Gew.-% |
| Aluminium: | 5 bis 45 Gew.-% |
| Chrom: | 0 bis 3 Gew.-% |
| Nickel: | 0 bis 7 Gew.-% |

oder
2. Variante

| | |
|---|---|
| Cobalt: | 55 bis 90 Gew.-% |
| Aluminium: | 5 bis 44,5 Gew.-% |
| Chrom: | 0,5 bis 5 Gew.-% |

oder
3. Variante

| | |
|---|---|
| Cobalt: | 55 bis 88 Gew.-% |
| Aluminium: | 5 bis 44,5 Gew.-% |
| Nickel: | 0,5 bis 7 Gew.-% |

oder
4. Variante

| | |
|---|---|
| Cobalt: | 55 bis 85 Gew.-% |
| Aluminium: | 5 bis 43,5 Gew.-% |
| Chrom: | 0,5 bis 3 Gew.-% |
| Nickel: | 1 bis 7 Gew.-% |

5. Variante

| | |
|---|---|
| Cobalt: | 57 bis 84 Gew.-% |
| Aluminium: | 10 bis 40 Gew.-% |
| Chrom: | 1 bis 2 Gew.-% |
| Nickel: | 2 bis 4 Gew.-% |

Gesamtheit bedeutet, dass bei der Zusammensetzung nicht zwischen dem Gehalt der Metalle auf der Oberfläche und in der aktivierten Schicht und im Kern der Katalysator-Partikel unterschieden wird, sondern Alles zusammen addiert und berechnet wird.

Der Katalysator liegt in Form von unregelmäßigen Partikeln also als Granulat vor.

Zusätzlich weist der erfindungsgemäße Katalysator nach der Aktivierung die folgenden Partikelgrößen auf:
Im Allgemeinen kann der Katalysator, also die Granulat-Teilchen Partikelgrößen von 1 bis 8 Millimeter (mm) aufweisen.

In einer ersten bevorzugten Variante der Erfindung variieren die Partikelgrößen des Katalysators, also die Granulat-Teilchen von 2,5 bis 6 Millimeter (mm).

In einer zweiten bevorzugten Variante der Erfindung variieren die Partikelgrößen des Katalysators, also die Granulat-Teilchen von 3 bis 7 Millimeter (mm).

In einer dritten bevorzugten Variante der Erfindung variieren die Partikelgrößen des Katalysators, also die Granulat-Teilchen von 2 bis 5 Millimeter (mm).

Die angegebenen Partikelgrößen können auch innerhalb der Bereiche eine statistische Verteilung der Größe aufweisen. Dabei sind enge Verteilungen als auch breite Verteilungen erfindungsgemäß.

Die Bestimmung der Partikelgrößen wird in der DIN ISO 9276-1 (September 2004) und 9276-2 (Februar 2006) und 9276-4 (Februar 2006) und 9276-6 (Januar 2012) beschrieben. Außerdem findet man genaue Angaben zur Definition von Partikelgrößen, der Verteilung von Partikelgrößen und der Messung von Partikelgrößen in HORIBA® Scientific, A GUIDEBOOK TO PARTICLE SIZE ANALYSIS, 2012, von HORIBA® Instruments, Inc, Irvine, USA.

Erfindungsgemäß kann die Verteilung der Partikelgrößen und die Messung der Partikelgrößen durch Laserverfahren (ISO 13320, 2012), Lichtverfahren oder Bildverfahren bestimmt werden.

Bevorzugt wird der erfindungsgemäße Katalysator durch Siebung der hergestellten Granulate erhalten. Dabei werden sogenannte Siebfraktionen hergestellt. Dabei können einzelne Siebfraktionen gemischt werden, oder es wird ein Katalysator durch einmalige oder mehrmalige Siebung erhalten. Die so hergestellten Katalysatoren weisen bei den Partikelgrößen eine statistische Verteilung auf, üblicherweise in Form einer Gauß-Verteilung. Es sind symmetrische aber auch asymmetrische Verteilungen möglich.

In einer vierten bevorzugten Variante der Erfindung variieren die Partikelgrößen des Katalysators, also die Granulat-Teilchen mit einer statistischen Verteilung zwischen 2,5 bis 5,5 Millimeter (mm), und wobei bis zu 10% der Partikel oberhalb der genannten Obergrenze und bis zu 10% der Partikel unterhalb der genannten Untergrenze liegen können.

In einer fünften bevorzugten Variante der Erfindung variieren die Partikelgrößen des Katalysators, also die Granulat-Teilchen mit einer statistischen Verteilung zwischen 3,5 bis 6,5 Millimeter (mm), und wobei bis zu 10% der Partikel oberhalb der genannten Obergrenze und bis zu 10% der Partikel unterhalb der genannten Untergrenze liegen können.

In einer sechsten bevorzugten Variante der Erfindung variieren die Partikelgrößen des Katalysators, also die Granulat-Teilchen mit einer statistischen Verteilung zwischen 2 bis 5 Millimeter (mm), und wobei bis zu 10% der Partikel oberhalb der genannten Obergrenze und bis zu 10% der Partikel unterhalb der genannten Untergrenze liegen können.

In einer siebten bevorzugten Variante der Erfindung variieren die Partikelgrößen des Katalysators, also de Granulat-Teilchen mit einer statistischen Verteilung zwischen 3 bis 7 Millimeter (mm), und wobei bis zu 10% der Partikel oberhalb der genannten Obergrenze und bis zu 10% der Partikel unterhalb der genannten Untergrenze liegen können.

Geeignete Methoden und Beschreibungen zur Siebanalyse sind beschrieben in:DIN 66165-1:1987-04 Partikelgrößenanalyse; Siebanalyse; Grundlagen, und in DIN 66165-2:1987-04 Partikelgrößenanalyse; Siebanalyse; Durchführung.
Paul Schmidt, Rolf Körber, Matthias Coppers: Sieben und Siebmaschinen: Grundlagen und Anwendung. Wiley-VCH Verlag, 2003, ISBN 9783527302079, Kapitel 4.4: Analysesiebung. Jörg Hoffmann: Handbuch der Messtechnik. Hanser Verlag, 2007, ISBN 978-3-446-40750-3, Kapitel 3.12.16.2.1.

Der erfindungsgemäße Katalysator weist besonders bevorzugt nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle:

| | |
|---|---|
| Cobalt: | 57 bis 84 Gew.-% |
| Aluminium: | 10 bis 40 Gew.-% |
| Chrom: | 1 bis 2 Gew.-% |
| Nickel: | 2 bis 4 Gew.-% |

und mit
Partikelgrößen des Katalysators, also die Granulat-Teilchen mit einer statistischen Verteilung zwischen 2,5 bis 5,5 Millimeter (mm),
oder
Partikelgrößen des Katalysators, also die Granulat-Teilchen mit einer statistischen Verteilung zwischen 3,5 bis 6,5 Millimeter (mm),
oder
Partikelgrößen des Katalysators, also die Granulat-Teilchen mit einer statistischen Verteilung zwischen 2 bis 5 Millimeter (mm),
oder
Partikelgrößen des Katalysators, also die Granulat-Teilchen mit einer statistischen Verteilung zwischen 3 bis 7 Millimeter (mm),
und wobei bis zu 10% der Partikel oberhalb der genannten Obergrenze und bis zu 10% der Partikel unterhalb der genannten Untergrenze liegen können.

Allgemeine Methode zur Herstellung des Katalysators:

### a) Herstellung der Legierung

Die Herstellung der Legierung erfolgt thermisch, zum Beispiel in einem Induktionsofen. Es werden dabei die Metalle geschmolzen und eine Legierung erhalten. Die fertige Schmelze wird für die Weiterverarbeitung zum Beispiel zu Barren gegossen.

### b) Herstellung der Granulate

Die Legierung wird in geeigneten Geräten zu Granulaten verarbeitet, zum Beispiel über einen Backenbrecher vorzerkleinert und über eine Walzenmühle weiter gemahlen. Durch einen Siebschritt wird die gewünschte Größenverteilung der Granulate durch die Wahl der entsprechenden Siebe erhalten (z. B. 3 bis 7 mm).

### c) Aktivierung des Katalysators

Die Aktivierung des Katalysators erfolgt in geeigneten Apparaturen. Es können dabei organische oder anorganische Basen eingesetzt werden. Bevorzugt wird eine Lauge (z. B. Natronlauge), verwendet, wobei durch einen exothermen Vorgang ein Teil des Aluminiums unter Bildung von Wasserstoff und Aluminatlauge aus der Legierung herausgelöst wird. Die Konzentration der Lauge kann zwischen 5 und 30 Gew.-% liegen und die Reaktionstemperatur zwischen 50 und 120 °C. Der Grad der Aktivierung wird über die Temperatur und die Reaktionszeit bestimmt. Dabei ist die Reaktionszeit variabel und ist abhängig von den Reaktionsbedingungen und dem gewünschten Grad der Aktivierung. Nach der Aktivierung wird der Katalysator mit Wasser gewaschen und anschließend unter Wasser gelagert.
Andere Zusammensetzungen können im Herstellungsschritt a) durch die entsprechende Wahl der Metallmengen analog produziert werden.

Bevorzugt wird der Katalysator in der beschriebenen Reihenfolge hergestellt. Die Aktivierung des Katalysators kann aber auch vor der Herstellung der Granulate erfolgen.

Zur Erhöhung der Aktivität, Selektivität und/oder Standzeit können die Katalysatoren zusätzlich Dotiermetalle oder andere Modifizierungsmittel enthalten. Typische Dotiermetalle sind z. B. Mo, Fe, Ag, V, Ga, In, Bi, Ti, Zr und Mn sowie die seltenen Erden, allein oder in Mischungen. Typische Modifizierungsmittel sind z. B. solche, mit denen die Säure-Base-Eigenschaften der Katalysatoren beeinflusst werden können, bevorzugt Alkali- und Erdalkalimetalle bzw. deren Verbindungen, bevorzugt Mg- und Li-Verbindungen. Für den Fall, dass derartige Verbindungen enthalten sind, in einer Menge von maximal ca. 5 Gew.-%, reduziert sich der Anteil der oben genannten Metall Co und Al sowie gegebenenfalls Cr und Ni im Katalysator entsprechend, wobei die Anteile an Co und Al sowie gegebenenfalls Cr und Ni dann nach der Aktivierung sich zu mindestens 95 Gew.-% addieren, bezogen auf die enthaltenden Metalle.

### Durchführung der Hydrierung allgemein

Die Hydrierung erfolgt einstufig in Batch-Autoklaven oder Festbettreaktoren. Geeignete Reaktortypen sind z.B. Schachtöfen, Hordenreaktoren oder Rohrbündelreaktoren.

Der Prozess kann in diskontinuierlicher oder kontinuierlicher Fahrweise durchgeführt werden.

Die Hydrierung erfolgt üblicherweise bei Temperaturen zwischen 20 bis 200°C, bevorzugt 20 bis 150 °C, besonders bevorzugt 40 bis 130 °C, und Drücken von 0,3 bis 50 MPa, bevorzugt 0,3 bis 30 MPa, besonders bevorzugt 5 bis 15 MPa. Die Bereiche für Druck und Temperatur sind beliebig kombinierbar. Die Hydrierung kann in An- oder Abwesenheit von Lösungsmittel durchgeführt werden. Als Lösungsmittel eignen sich u.a. Ether, cyclische und lineare Kohlenwasserstoffe und Alkohole, bevorzugt werden Methanol, Ethanol oder THF verwendet.

Der für die Hydrierung erforderliche Wasserstoff kann dem Reaktor entweder im Überschuss, beispielsweise mit bis zu 10000 Moläquivalenten, zugeführt werden, oder nur in einer solchen Menge, dass der durch Reaktion verbrauchte Wasserstoff sowie der Teil des Wasserstoffs, der eingelöst im Produktstrom den Reaktor verlässt, nachgeführt wird. Bei kontinuierlicher Fahrweise kann der Wasserstoff im Gleich- oder Gegenstrom zugeführt werden.

Das erforderliche Volumen der einzusetzenden Hydrierkatalysatoren richtet sich nach dem vom Betriebsdruck, der Temperatur, der Konzentration und der Katalysatoraktivität abhängigen LHSV-Wert, (liquid hourly space velocity) der eingehalten werden muss, um eine möglichst vollständige Hydrierung des eingesetzten IPN zu gewährleisten. Üblicherweise liegt der LHSV-Wert bei der Verwendung des bevorzugt einzusetzenden Gemisches aus IPN und Wasserstoff zwischen 0,1 und 8 pro Liter Katalysator und Stunde, bevorzugt zwischen 1 und 4 I_{Lsg} I_{Kat}⁻¹ h⁻¹.

Bevorzugt wird die Hydrierung kontinuierlich in Festbettreaktoren durchgeführt, besonders bevorzugt kontinuierlich in Festbettreaktoren, die in Rieselbettfahrweise oder Sumpffahrweise betrieben werden.

Das die Hydrierung verlassende Reaktionsgemisch wird mit den üblichen Methoden weiter aufgereinigt, um ein IPAA mit der gewünschten Qualität zu erhalten. Dabei können alle gängigen Trennmethoden wie z.B. Destillation, Entspannungsverdampfung, Kristallisation, Extraktion, Sorption, Permeation, Phasentrennung oder Kombinationen aus den genannten zum Einsatz kommen. Die Aufreinigung kann kontinuierlich, absatzweise, ein- oder mehrstufig, im Vakuum oder unter Druck durchgeführt werden.
Bevorzugt wird die Aufreinigung durch Destillation unter Druck und/oder im Vakuum in mehreren Schritten erreicht. Hierfür lassen sich beliebige Destillationskolonnen mit und ohne Einbauten wie z.B. Dephlegmatoren, Trennwänden, ungeordnete Einbauten oder Schüttungen, geordnete Einbauten oder Packungen, Böden mit oder ohne Zwangsführung verwenden.

### Beispiele

### Beispiel 1: Herstellung des erfindungsgemäßen Katalysators

### a) Herstellung der Legierung

Die Herstellung der Legierung erfolgte in einem Induktionsofen. Es wurden dabei die Metalle in den entsprechenden Mengen bei 1500°C geschmolzen. Die fertige Schmelze wurde für die Weiterverarbeitung zu Barren gegossen.

### b) Herstellung der Granulate

Die Legierungsbarren wurden über einen Backenbrecher vorzerkleinert und über eine Walzenmühle weiter gemahlen. Durch einen Siebschritt wurde die gewünschte Größenverteilung der Granulate durch die Wahl der entsprechenden Siebe erhalten.

### c) Aktivierung des Katalysators

Die Aktivierung des Katalysators erfolgte in einer Standard-Glaslaborapparatur, z.B. einem Becherglas. Zu den Granulaten wurde unter Rühren eine wässrige Lauge (z.B. Natronlauge) gegeben. Die Granulate befanden sich während der Aktivierung in einem Katalysatorkorb. Durch den exothermen Vorgang der Aktivierung wird ein Teil des Aluminiums unter Bildung von Wasserstoff und Natriumaluminatlauge aus der Legierung herausgelöst. Die Konzentration der eingesetzten Lauge lag bei 20 Gew.-% und die Reaktionstemperatur bei 90°C. Der Grad der Aktivierung wurde über die Reaktionszeit bestimmt. Nach der Aktivierung wurde der Katalysator mit Wasser gewaschen und anschließend unter Wasser gelagert.
Der eingesetzte Katalysator weist nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle:

| | |
|---|---|
| Cobalt: | 75,9 Gew.-% |
| Aluminium: | 20,0 Gew.-% |
| Chrom: | 1,5 Gew.-% |
| Nickel: | 2,6 Gew.-% |

Es wurde eine Siebfraktion eingesetzt mit Partikelgrößen des Katalysators, also die Granulat-Teilchen, mit einer statistischen Verteilung zwischen 2,0 bis 5,0 Millimeter (mm), wobei bis zu 10% der Partikel oberhalb der genannten Obergrenze und bis zu 10% der Partikel unterhalb der genannten Untergrenze liegen können.

### Beispiel 2:

Der erfindungsgemäße Katalysator wurde bei der Herstellung von 3-Aminomethyl-3,5,5-Trimethylcyclohexanol (Isophoronaminoalkohol, IPAA) aus 3-Cyano-3,5,5-Trimethylcyclohexanon (Isophoronnitril, IPN) in einem diskontinuierlichen Verfahren in einem Laborautoklaven auf seine katalytische Wirksamkeit getestet.

Für die Durchführung der einzelnen Experimente wurde die Reaktionslösung (5 Gew.-% IPN in 95 Gew.-% THF) im Reaktor vorgelegt. 150 ml des Katalysators befanden sich dabei in einem speziellen Katalysatorkorb. Nach dem Aufheizen auf die gewünschte Reaktionstemperatur wurde der gewünschte Reaktionsdruck durch Zugabe von Wasserstoff eingestellt. Die Untersuchung der Hydrierung von IPN mit den erfindungsgemäßen Katalysatoren erfolgte bei einer Temperatur von 60 und 80 °C bei einem Druck von 50 und 100 bar.
Die Reaktionsmischung wurde auf IPN, IPAA und entsprechende Nebenkomponenten gaschromatographisch untersucht. Die Ergebnisse sind in Tabelle 1 aufgeführt.

**Tabelle 1**

| Temperatur | Druck | Reaktionszeit | Ausbeute IPAA/GC-% | Umsatz / % |
|---|---|---|---|---|
| 60°C | 50 bar | 4h | 96,4 | 100 |
| 60°C | 100 bar | 3h | 97,1 | 100 |
| 80°C | 50 bar | 3h | 96,1 | 100 |

### Beispiel A (Vergleichsbeispiel):

Die Vergleichsbeispiele mit einem kommerziellen Referenzkatalysator wurden analog zur Beschreibung in Beispiel 2 durchgeführt. Als Referenzkatalysator wurden 150ml eines kommerziellen, geträgerten Cobalt- **Fischer-Tropsch**-Katalysatorformkörpers (Pellets) eingesetzt. Die Reaktionsmischung wurde auf IPN, IPAA und entsprechende Nebenkomponenten gaschromatographisch untersucht. Die Ergebnisse sind in Tabelle 2 aufgeführt.

**Tabelle 2**

| Temperatur | Druck | Reaktionszeit | Ausbeute IPAA/GC-% | Umsatz / % |
|---|---|---|---|---|
| 60°C | 50 bar | 5h | 40,4 | 85,1 |
| 60°C | 100 bar | 5h | 43,1 | 95,4 |
| 80°C | 50 bar | 5h | 44,2 | 98,2 |

Wie der Fachmann anhand der Tabelle 2 erkennen kann, ist die Ausbeute im Falle des kommerziellen Referenzkatalysators bei längerer Reaktionszeit deutlich geringer, ebenso wie die erzielten Umsätze.

## Patentansprüche

1. Verfahren zur einstufigen Herstellung von 3-(Aminomethyl)-3,5,5-trimethylcyclohexanol, durch Hydrierung von Isophoronnnitril mit Wasserstoff an einem Katalysator, in An- oder Abwesenheit von Lösungsmittel,
wobei der Katalysator die folgenden Eigenschaften aufweist:
I. Der Katalysator weist nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle:
| | |
|---|---|
| Cobalt: | 55 bis 95 Gew.-% |
| Aluminium: | 5 bis 45 Gew.-% |
| Chrom: | 0 bis 3 Gew.-% |
| Nickel: | 0 bis 7 Gew.-% |
und
II. Der Katalysator liegt in Form von unregelmäßigen Partikeln als Granulat vor und weist nach der Aktivierung Partikelgrößen von 1 bis 8 Millimeter (mm) auf.

2. Verfahren zur Herstellung von 3-(Aminomethyl)-3,5,5-trimethylcyclohexanol nach Anspruch 1, **dadurch gekennzeichnet, dass**
I. Der Katalysator nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle, aufweist:
| | |
|---|---|
| Cobalt: | 55 bis 90 Gew.-% |
| Aluminium: | 5 bis 44,5 Gew.-% |
| Chrom: | 0,5 bis 5 Gew.-% |

3. Verfahren zur Herstellung von 3-(Aminomethyl)-3,5,5-trimethylcyclohexanol nach Anspruch 1, **dadurch gekennzeichnet, dass**
I. Der Katalysator nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle, aufweist:
| | |
|---|---|
| Cobalt: | 55 bis 88 Gew.-% |
| Aluminium: | 5 bis 44,5 Gew.-% |
| Nickel: | 0,5 bis 7 Gew.-% |

4. Verfahren zur Herstellung von 3-(Aminomethyl)-3,5,5-trimethylcyclohexanol nach Anspruch 1, **dadurch gekennzeichnet, dass**
I. Der Katalysator nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle, aufweist:
| | |
|---|---|
| Cobalt: | 55 bis 85 Gew.-% |
| Aluminium: | 5 bis 43,5 Gew.-% |
| Chrom: | 0,5 bis 3 Gew.-% |
| Nickel: | 1 bis 7 Gew.-% |

5. Verfahren zur Herstellung von 3-(Aminomethyl)-3,5,5-trimethylcyclohexanol nach Anspruch 1, **dadurch gekennzeichnet, dass**
I. Der Katalysator nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle, aufweist:
| | |
|---|---|
| Cobalt: | 57 bis 84 Gew.-% |
| Aluminium: | 10 bis 40 Gew.-% |
| Chrom: | 1 bis 2 Gew.-% |
| Nickel: | 2 bis 4 Gew.-% |

6. Verfahren zur Herstellung von 3-(Aminomethyl)-3,5,5-trimethylcyclohexanol nach mindestens einem der vorherigen Ansprüche 1-5, **dadurch gekennzeichnet, dass** die Partikelgrößen des Katalysators, also die Granulat-Teilchen von 2,5 bis 6 Millimeter (mm) variieren,
oder
die Partikelgrößen des Katalysators, also die Granulat-Teilchen von 3 bis 7 Millimeter (mm) variieren,
oder
die Partikelgrößen des Katalysators, also die Granulat-Teilchen von 2 bis 5 Millimeter (mm) variieren.

7. Verfahren zur Herstellung von 3-(Aminomethyl)-3,5,5-trimethylcyclohexanol nach mindestens einem der vorherigen Ansprüche 1-5, **dadurch gekennzeichnet, dass** der Katalysator durch Siebung der hergestellten Granulate erhalten wird.

8. Verfahren zur Herstellung von 3-(Aminomethyl)-3,5,5-trimethylcyclohexanol nach Anspruch 7, **dadurch gekennzeichnet, dass** der Katalysator durch Siebung der hergestellten Granulate erhalten wird, und
die Partikelgrößen des Katalysators, also die Granulat-Teilchen mit einer statistischen Verteilung zwischen 2,5 bis 5,5 Millimeter (mm), liegen, oder
die Partikelgrößen des Katalysators, also die Granulat-Teilchen mit einer statistischen Verteilung zwischen 3,5 bis 6,5 Millimeter (mm), liegen, oder
die Partikelgrößen des Katalysators, also die Granulat-Teilchen mit einer statistischen Verteilung zwischen 2 bis 5 Millimeter (mm), liegen, oder
die Partikelgrößen des Katalysators, also de Granulat-Teilchen mit einer statistischen Verteilung zwischen 3 bis 7 Millimeter (mm),
und wobei bis zu 10% der Partikel oberhalb der genannten Obergrenze und bis zu 10% der Partikel unterhalb der genannten Untergrenze liegen können.

9. Verfahren zur Herstellung von 3-(Aminomethyl)-3,5,5-trimethylcyclohexanol nach Anspruch 1, **dadurch gekennzeichnet, dass** der Katalysator nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle, aufweist:
| | |
|---|---|
| Cobalt: | 57 bis 84 Gew.-% |
| Aluminium: | 10 bis 40 Gew.-% |
| Chrom: | 1 bis 2 Gew.-% |
| Nickel: | 2 bis 4 Gew.-% |
und mit
Partikelgrößen des Katalysators, also die Granulat-Teilchen mit einer statistischen Verteilung zwischen 2,5 bis 5,5 Millimeter (mm),
oder
Partikelgrößen des Katalysators, also die Granulat-Teilchen mit einer statistischen Verteilung zwischen 3,5 bis 6,5 Millimeter (mm),
oder
Partikelgrößen des Katalysators, also die Granulat-Teilchen mit einer statistischen Verteilung zwischen 2 bis 5 Millimeter (mm),
oder
Partikelgrößen des Katalysators, also die Granulat-Teilchen mit einer statistischen Verteilung zwischen 3 bis 7 Millimeter (mm),
wobei bis zu 10% der Partikel oberhalb der genannten Obergrenze und bis zu 10% der Partikel unterhalb der genannten Untergrenze liegen können.

10. Verfahren zur Herstellung von 3-(Aminomethyl)-3,5,5-trimethylcyclohexanol nach mindestens einem der vorherigen Ansprüche, **dadurch gekennzeichnet, dass** die Katalysatoren zusätzlich Dotiermetalle enthalten, bevorzugt ausgewählt aus Mo, Fe, Ag, V, Ga, In, Bi, Ti, Zr und Mn, sowie den seltenen Erden.

11. Verfahren zur Herstellung von 3-(Aminomethyl)-3,5,5-trimethylcyclohexanol nach mindestens einem der vorherigen Ansprüche, **dadurch gekennzeichnet, dass** Katalysatoren Modifizierungsmittel enthalten, bevorzugt Alkali- und Erdalkalimetalle bzw. deren Verbindungen, bevorzugt Mg- und Li-Verbindungen.

12. Verfahren zur Herstellung von 3-(Aminomethyl)-3,5,5-trimethylcyclohexanol nach mindestens einem der vorherigen Ansprüche, **dadurch gekennzeichnet, dass** die Hydrierung bei einer Temperatur von 20 bis 200°C und bei einem Druck von 0,3 bis 50 MPa durchgeführt wird.

13. Verfahren zur Herstellung von 3-(Aminomethyl)-3,5,5-trimethylcyclohexanol nach mindestens einem der vorherigen Ansprüche, **dadurch gekennzeichnet, dass** die Hydrierung bei einer Temperatur von 40 bis 130 °C durchgeführt wird.

14. Verfahren zur Herstellung von 3-(Aminomethyl)-3,5,5-trimethylcyclohexanol nach mindestens einem der vorherigen Ansprüche, **dadurch gekennzeichnet, dass** die Hydrierung bei einem Druck von 5 bis 15 MPa durchgeführt wird.

15. Verfahren zur Herstellung von 3-(Aminomethyl)-3,5,5-trimethylcyclohexanol nach mindestens einem der vorherigen Ansprüche, **dadurch gekennzeichnet, dass** die Hydrierung einstufig in Batch-Autoklaven oder Festbettreaktoren erfolgt.

16. Verfahren zur Herstellung von 3-(Aminomethyl)-3,5,5-trimethylcyclohexanol nach mindestens einem der vorherigen Ansprüche, **dadurch gekennzeichnet, dass** die Hydrierung einstufig in Schachtöfen, Hordenreaktoren oder Rohrbündelreaktoren durchgeführt wird.

17. Verfahren zur Herstellung von 3-(Aminomethyl)-3,5,5-trimethylcyclohexanol nach mindestens einem der vorherigen Ansprüche, **dadurch gekennzeichnet, dass** der für die Hydrierung erforderliche Wasserstoff dem Reaktor entweder im Überschuss, bevorzugt mit bis zu 10000 Moläquivalenten, zugeführt wird, oder in einer solchen Menge, dass der durch Reaktion verbrauchte Wasserstoff sowie der Teil des Wasserstoffs, der eingelöst im Produktstrom den Reaktor verlässt, nachgeführt wird.

18. Verfahren zur Herstellung von 3-(Aminomethyl)-3,5,5-trimethylcyclohexanol nach mindestens einem der vorherigen Ansprüche, **dadurch gekennzeichnet, dass** Hydrierung kontinuierlich in Festbettreaktoren erfolgt.

19. Verfahren zur Herstellung von 3-(Aminomethyl)-3,5,5-trimethylcyclohexanol nach mindestens einem der vorherigen Ansprüche, **dadurch gekennzeichnet, dass** die Hydrierung kontinuierlich in Festbettreaktoren, die in Rieselbettfahrweise oder Sumpffahrweise betrieben werden, durchgeführt wird.

20. Verfahren zur Herstellung von 3-(Aminomethyl)-3,5,5-trimethylcyclohexanol nach mindestens einem der vorherigen Ansprüche, **dadurch gekennzeichnet, dass** das die Hydrierung verlassende Reaktionsgemisch einstufig oder mehrstufig aufgereinigt wird, und das 3-(Aminomethyl)-3,5,5-trimethylcyclohexanolerhalten wird.

21. Verwendung eines Katalysators zur Herstellung von 3-(Aminomethyl)-3,5,5-trimethylcyclohexanol, **dadurch gekennzeichnet, dass**
wobei der Katalysator die folgenden Eigenschaften aufweist:
I. Der Katalysator weist nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle:
| | |
|---|---|
| Cobalt: | 55 bis 95 Gew.-% |
| Aluminium: | 5 bis 45 Gew.-% |
| Chrom: | 0 bis 3 Gew.-% |
| Nickel: | 0 bis 7 Gew.-% |
und
II. Der Katalysator liegt in Form von unregelmäßigen Partikeln als Granulat vor und weist nach der Aktivierung Partikelgrößen von 1 bis 8 Millimeter (mm) auf.
